# EUROPEAN PATENT APPLICATION

(11) **EP 4 803 640 A1**
(43) Date of publication of application: **09.09.2026**
(21) Application number: 24885947.2
(22) Date of filing: 17.06.2024
(51) Int. Cl.: C12Q 1/689

(54) **COMPOSITION FOR DETECTING STAPHYLOCOCCUS EPIDERMIDIS CICARIA STRAIN, AND USE THEREOF**

(30) Priority: 02.11.2023 KR 20230150290
(71) Applicant: Cosmax, Inc., Hwaseong-si, Gyeonggi-do 18622 (KR)
(72) Inventor: LEE, Ha Eun, Seongnam-si, Gyeonggi-do 13486 (KR); LEE, Dong Geol, Seongnam-si, Gyeonggi-do 13486 (KR); KANG, Seung Hyun, Seoul 06285 (KR)
(74) Representative: Lavoix
(86) International application number: PCT/KR2024/008338
(87) International publication number: WO 2025/095265

(57) **Abstract**

Provided are a composition for detecting a *Staphylococcus epidermidis* CICARIA strain, a primer set, and a detection method using the same. The composition can rapidly and accurately detect or identify the *Staphylococcus epidermidis* CICARIA strain with high detection specificity at a strain or species level.

## Description

### Technical Field

The present disclosure relates to a composition for identifying microorganisms at a specific strain level and use thereof.

### Background Art

The skin is the largest human organ in contact with the external environment. The skin protects the body from external stimuli and has an essential function of preventing moisture from evaporating from the skin. Along with other body organs such as the intestines, various microorganisms, such as bacteria, fungi, mites, and viruses, inhabit the skin while maintaining specialized functions. Thus, microorganisms present on the skin and the entirety of their genetic information (genome) are defined as the skin microbiome.

Skin microbiome research has become possible following the development of Next Generation Sequencing (NGS) analysis methods. In particular, when 16S rRNA gene primers are utilized, the bacterial composition of microbiota can be cataloged and processed.

Although such a 16S rRNA gene-based NGS analysis method has an advantage of being able to detect all microorganisms in a specific environment, the method has limitations in that, to date, it takes at least one week and specific strains cannot be detected using the 16S rRNA gene. In addition, there is a disadvantage in that detecting a specific strain using NGS involves high costs.

(Patent Document 1) KR 10-2305609 B1

### Disclosure of Invention

### Technical Problem

The present disclosure relates to a molecular marker based on whole genome sequence (WGS) data, capable of identifying a *Staphylococcus epidermidis* CICARIA strain at a strain or species level. By using the molecular marker, the *Staphylococcus epidermidis* CICARIA strain can be relatively quickly and accurately identified through a nucleic acid amplification reaction such as polymerase chain reaction (PCR) and the like.

One aspect is to provide a composition for detecting a *Staphylococcus epidermidis* CICARIA strain deposited under Accession No. KCCM12559P, including an agent capable of specifically detecting a polynucleotide consisting of a sequence at positions 3,487 to 3,608 of SEQ ID NO: 1 or a complementary polynucleotide thereof.

Another aspect is to provide a primer set for detecting a *Staphylococcus epidermidis* CICARIA strain deposited under Accession No. KCCM12559P, consisting of a forward primer set forth in SEQ ID NO: 2 and a reverse primer set forth in SEQ ID NO: 3.

Another aspect is to provide a kit for detecting a *Staphylococcus epidermidis* CICARIA strain deposited under Accession No. KCCM12559P, including the composition according to one aspect.

Another aspect is to provide a method of detecting a *Staphylococcus epidermidis* CICARIA strain deposited under Accession No. KCCM12559P, using the composition, the primer set, or the kit according to one aspect.

### Solution to Problem

One aspect provides a composition for detecting a *Staphylococcus epidermidis* CICARIA strain deposited under Accession No. KCCM12559P, including an agent capable of specifically detecting a polynucleotide consisting of a sequence at positions 3,487 to 3,608 of SEQ ID NO: 1 or a complementary polynucleotide thereof.

The *Staphylococcus epidermidis* CICARIA strain deposited under Accession No. KCCM12559P is disclosed in Korean Patent No. 10-2305609.

SEQ ID NO: 1 may be a complete sequence of plasmid DNA of the *Staphylococcus epidermidis* CICARIA strain deposited under Accession No. KCCM12559P.

The polynucleotide consisting of the sequence at positions 3,487 to 3,608 of SEQ ID NO: 1 or the complementary polynucleotide thereof may be a partial sequence of the plasmid DNA of the *Staphylococcus epidermidis* CICARIA strain.

The polynucleotide consisting of the sequence at positions 3,487 to 3,608 of SEQ ID NO: 1 or the complementary polynucleotide thereof may be a whole genome sequence (WGS)-based molecular marker capable of identifying the *Staphylococcus epidermidis* CICARIA strain at a strain or species level. The term "molecular marker" refers to a molecule having a characteristic capable of identifying the presence or state of a certain organism or substance. For example, a specific DNA base sequence may be used as a molecular marker to determine the presence or absence of a specific strain. When the sequence at positions 3,487 to 3,608 of SEQ ID NO: 1 or a complementary sequence thereof is present in a nucleic acid sequence of a microorganism to be analyzed, the microorganism may be identified as the *Staphylococcus epidermidis* CICARIA strain.

In addition, since the polynucleotide consisting of the sequence at positions 3,487 to 3,608 of SEQ ID NO: 1 or the complementary polynucleotide thereof is a whole genome sequence (WGS)-based molecular marker, it has significantly superior detection specificity compared to a conventional 16S rRNA gene-based NGS analysis method that cannot detect a specific strain. Therefore, by utilizing the molecular marker, the *Staphylococcus epidermidis* CICARIA strain can be distinguished from microorganisms belonging to closely related strains or closely related species that are indistinguishable by general physiological or chemical differences.

In addition, when an agent capable of specifically detecting the polynucleotide consisting of the sequence at positions 3,487 to 3,608 of SEQ ID NO: 1 or the complementary polynucleotide thereof is used, a specific strain can be identified merely by detecting a molecular marker having a relatively short length (e.g., 122 bp), and thus time and cost may be significantly reduced compared to a conventional NGS analysis method that identifies a 16S rRNA gene having a length of hundreds to thousands of bp.

Therefore, the agent capable of specifically detecting the polynucleotide consisting of the sequence at positions 3,487 to 3,608 of SEQ ID NO: 1 or the complementary polynucleotide thereof may be an agent capable of detecting or identifying the *Staphylococcus epidermidis* CICARIA strain. The agent can specifically detect only the *Staphylococcus epidermidis* CICARIA strain, distinguishing the strain even from microorganisms that differ in detailed lineage from the *Staphylococcus epidermidis* CICARIA strain. Accordingly, the composition according to one aspect may be a composition for identifying the *Staphylococcus epidermidis* CICARIA strain deposited under Accession No. KCCM12559P.

As long as the agent is a substance capable of specifically binding to the polynucleotide or complementary polynucleotide thereof, the type of the agent is not limited. The agent may be a primer or a probe that specifically binds to the polynucleotide or complementary polynucleotide thereof, but is not limited thereto.

The term "primer" is a short single-stranded oligonucleotide that serves as a starting point from which another polymer strand complementary to a template is produced during DNA synthesis. The primers used in a nucleic acid amplification reaction may include a primer set consisting of a forward primer and a reverse primer. The length of the primer may be 15 to 40 nt, 15 to 30 nt, 15 to 25 nt, 18 to 40 nt, 18 to 30 nt, or 18 to 25 nt, but is not limited thereto.

When a nucleic acid amplification reaction is performed using primers that specifically bind to the polynucleotide consisting of the sequence at positions 3,487 to 3,608 of SEQ ID NO: 1 or the complementary polynucleotide thereof, the *Staphylococcus epidermidis* CICARIA strain can be specifically identified depending on whether an amplification product is detected.

In an embodiment, the primers may be a primer set consisting of a forward primer set forth in SEQ ID NO: 2 and a reverse primer set forth in SEQ ID NO: 3.

The term "probe" refers to a DNA or RNA fragment complementary to a specific base sequence of DNA or RNA, and is labeled with a radioactive element, a staining material, fluorescence, or the like, thereby enabling confirmation of the presence or absence of a specific base sequence. The length of the probe may be 10 to 1,000 bp, 10 to 200 bp, or 10 to 100 bp, but is not limited thereto.

When hybridization is performed using a probe that specifically binds to the polynucleotide consisting of the sequence at positions 3,487 to 3,608 of SEQ ID NO: 1 or the complementary polynucleotide thereof, the *Staphylococcus epidermidis* CICARIA strain can be specifically identified based on whether hybridization occurs.

In an embodiment, the probe may be an oligonucleotide consisting of a consecutive nucleotide sequence of 10 or more, 20 or more, 30 or more, 40 or more, 50 or more, or 100 or more nucleotides complementary to part or all of 'the polynucleotide consisting of the sequence at positions 3,487 to 3,608 of SEQ ID NO: 1 or the complementary polynucleotide thereof.

In an embodiment, the probe may be an oligonucleotide consisting of a nucleotide sequence having a length of 122 nt complementary to 'the polynucleotide consisting of the sequence at positions 3,487 to 3,608 of SEQ ID NO: 1 or the complementary polynucleotide thereof.

The primers or probes may be chemically synthesized using known methods. The primers or probes may further include one or more labels selected from a radioactive element, a chromophore, a chemiluminophore, a fluorophore, and a magnetic particle, connected to a 5'-terminus or a 3'-terminus.

The term "label" or "detectable label" may refer to any chemical moiety bound to a nucleotide or a nucleotide polymer, and the binding may be a covalent bond or a noncovalent bond. The detectable labels are known in the art. Non-limiting examples of the detectable label include radioactive elements such as ³²P or ³⁵S; linker molecules such as biotin, avidin, streptavidin, horseradish peroxidase (HRP), protein A, protein G, an antibody or a fragment thereof, a FLAG tag, and a myc tag; enzymes such as peroxidase and luciferase; an electron donor and an acceptor; dyes such as Cy5 and Cy3; and magnetic particles (MP) such as iron oxide nanoparticles and gold nanoparticles.

Another aspect provides a primer set for detecting a *Staphylococcus epidermidis* CICARIA strain deposited under Accession No. KCCM12559P, consisting of a forward primer set forth in SEQ ID NO: 2 and a reverse primer set forth in SEQ ID NO: 3.

The forward primer set forth in SEQ ID NO: 2 has a length of 23 bp, a melting temperature (Tm) of about 60 °C, and a GC% of about 43 %.

The reverse primer set forth in SEQ ID NO: 3 has a length of 22 bp, a Tm of about 60 °C, and a GC% of about 50 %.

When a nucleic acid amplification reaction is performed using the primer set, an amplification product having a size of 122 bp can be detected only when the *Staphylococcus epidermidis* CICARIA strain is present.

In one example, it was confirmed that when PCR was performed using the primer set of SEQ ID NO: 2 and SEQ ID NO: 3 under a single condition having the same annealing temperature and time, no PCR product was synthesized for a *Staphylococcus caprae* 74 strain, a *Staphylococcus capitis* subsp. *capitis* 24 strain, and a *Staphylococcus capitis* subsp. *urealyticus* SH2 strain belonging to the same genus, while a PCR product was synthesized only in the *Staphylococcus epidermidis* CICARIA strain. These results confirmed that even when strains of the same genus are present in a sample, only the Staphylococcus epidermidis CICARIA strain can be specifically and unambiguously detected and identified at a species level.

In one example, it was confirmed that when PCR was performed using the primer set of SEQ ID NO: 2 and SEQ ID NO: 3 under a single condition having the same annealing temperature and time, no PCR product was synthesized for the *Staphylococcus epidermidis* KACC13234 strain and the *Staphylococcus epidermidis* KCTC1917 strain, which belong to the same species, while a PCR product was synthesized only in the *Staphylococcus epidermidis* CICARIA strain. These results confirmed that even when strains of the same species are present in a sample, only the *Staphylococcus epidermidis* CICARIA strain can be specifically and unambiguously detected and identified at a strain level.

Another aspect provides a kit for detecting a *Staphylococcus epidermidis* CICARIA strain deposited under Accession No. KCCM12559P, including the composition according to one aspect.

The kit may further include reagents required for a nucleic acid amplification reaction.

Any nucleic acid amplification technology known in the art may be used without limitation as the nucleic acid amplification reaction. The nucleic acid amplification reaction may be, for example, polymerase chain reaction (PCR). The PCR may include, but is not limited to, real-time PCR, reverse transcriptase polymerase chain reaction (RT-PCR), quantitative real-time polymerase chain reaction (qRT-PCR), and the like. Accordingly, the kit may be a PCR kit.

The reagents required for the nucleic acid amplification reaction may include DNA polymerase, dNTPs, a buffer solution, and the like.

The kit may further include a user guide describing optimal reaction performance conditions.

Another aspect provides a method of detecting the *Staphylococcus epidermidis* CICARIA strain deposited under Accession No. KCCM12559P, using the composition, the primer set, or the kit according to one aspect.

The detection may be performed by one or more methods selected from PCR analysis, DNA sequencing, hybridization by microarray, DNA amplification fingerprinting (DAF), Northern blot, and Southern blot, but is not limited thereto.

In an embodiment, the method may include: isolating nucleic acids from a sample;
performing a nucleic acid amplification reaction using the isolated nucleic acids as templates and the primer set according to one aspect; and
detecting an amplification product generated by the nucleic acid amplification reaction.

The sample may be a strain to be analyzed or a substance expected to contain the strain.

The nucleic acids may be DNA or RNA. The nucleic acids may be plasmid DNA (pDNA), genomic DNA (gDNA), or a mixture of plasmid DNA and genomic DNA. The genomic DNA is chromosomal DNA, and the plasmid DNA is extrachromosomal DNA. In an embodiment, the nucleic acids may be plasmid DNA. In another embodiment, the nucleic acids may be a mixture of plasmid DNA and genomic DNA.

In one example, it was confirmed that when PCR was performed using the primer set of SEQ ID NO: 2 and SEQ ID NO: 3 and total DNA (including plasmid DNA and genomic DNA) of each of *Staphylococcus epidermidis* CICARIA strain and closely related microorganisms as templates, a PCR product was synthesized only in the CICARIA strain. These results confirmed that even when a mixture of plasmid DNA and genomic DNA is used as a template, only the *Staphylococcus epidermidis* CICARIA strain can be specifically detected and identified.

Isolation of nucleic acids from the sample may be performed using a method known in the art. Nucleic acids may be isolated from the sample using a known method for isolating DNA. Nucleic acids may be isolated from the sample using a known method for isolating plasmid DNA. A non-limiting exemplary method for isolating plasmid DNA includes an alkaline lysis method and the like.

Any nucleic acid amplification technology known in the art may be used without limitation as the nucleic acid amplification reaction. The nucleic acid amplification reaction may be, for example, PCR. The PCR may include, but is not limited to, real-time PCR, reverse transcriptase polymerase chain reaction (RT-PCR), quantitative real-time polymerase chain reaction (qRT-PCR), and the like.

An annealing temperature of the nucleic acid amplification reaction may be 63 to 70 °C, 63 to 69 °C, 63 to 68 °C, 65 to 70 °C, 65 to 69 °C, 65 to 68 °C, 66 to 70 °C, 66 to 69 °C, 66 to 68 °C, or about 67 °C.

Detection of the amplification product may be performed through a DNA chip, gel electrophoresis, radioactivity measurement, fluorescence measurement, phosphorescence measurement, magnetic field measurement, or the like, but is not limited thereto.

In the detecting step, when an amplification product having a length of 122 bp is detected, the sample may be identified as the *Staphylococcus epidermidis* CICARIA strain deposited under Accession No. KCCM12559P, or the strain may be identified as being present in the sample.

Therefore, the method may be a method of identifying the *Staphylococcus epidermidis* CICARIA strain deposited under Accession No. KCCM12559P.

Redundant content is omitted in consideration of the complexity of the present disclosure, and terms not otherwise defined in the present disclosure have meanings commonly used in the technical field to which the present disclosure pertains.

### Advantageous Effects of Invention

Since the composition according to one aspect utilizes a whole genome sequence (WGS)-based molecular marker, the *Staphylococcus epidermidis* CICARIA strain can be rapidly and accurately detected or identified with high detection specificity at a strain or species level.

### Brief Description of Drawings

FIG. 1 shows NCBI Primer Blast verification results for a selected primer pair for detecting *Staphylococcus epidermidis* CICARIA.
FIG. 2 shows electrophoresis results confirming that the primer pair for detecting *Staphylococcus epidermidis* CICARIA specifically detects only a target microorganism.

### Best Mode for Carrying out the Invention

### Mode for the Invention

The present disclosure is described in more detail below through examples. However, these examples are provided for illustratively describing one or more embodiments, and the scope of the present disclosure is not limited to these examples.

### Example 1: Preparation of Primers

Primers capable of specifically detecting the *Staphylococcus epidermidis* CICARIA strain (Accession No.: KCCM12559P) were prepared.

First, whole genome sequences of microorganisms belonging to closely related strains or closely related species, which are indistinguishable by general physiological or chemical differences, were collected from the National Center for Biotechnology Information (NCBI). The target strain and comparative strains are shown in Table 1.

**[Table 1]**

| | **Genus** | **Species** | **Strain** |
|---|---|---|---|
| **Target Strain** | *Staphylococcus* | *epidermidis* | CICARIA |
| **Comparative Strain 1** | *Staphylococcus* | *epidermidis* | KACC13234 |
| **Comparative Strain 2** | *Staphylococcus* | *epidermidis* | KCTC1917 |
| **Comparative Strain 3** | *Staphylococcus* | *caprae* | 74 |
| **Comparative Strain 4** | *Staphylococcus* | *capitis* subsp. *capitis* | 24 |
| **Comparative Strain 5** | *Staphylococcus* | *capitis* subsp. *urealyticus* | SH2 |

After the whole genome sequence information of the strains in Table 1 was fragmented into units of 10 to 500 bp, numbers were assigned to the fragmented sequences using Linux commands. In addition, sequence information for the remaining regions was collected by excluding regions having the same sequence using NCBI's Nucleotide Blast. Thereafter, candidate primers were designed from regions containing rare sequences using a primer design system such as NCBI Primer Blast.

### Example 2: In-silico PCR of Candidate Primers

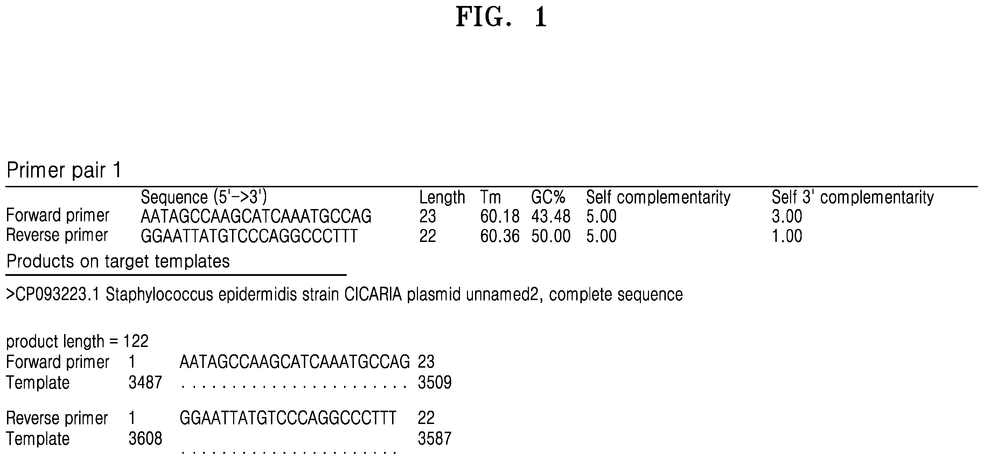

Prior to the preparation of the primers selected as candidates in Example 1, theoretical validation of the primers was performed through in-silico PCR using a server. Results confirming the finally selected primer pair using NCBI Primer Blast are shown in FIG. 1.

As a result, as shown in FIG. 1, the finally selected primer pair is capable of specifically detecting a 122 bp sequence at positions 3,487 to 3,608 of the complete sequence of the plasmid DNA (SEQ ID NO: 1) of the *Staphylococcus epidermidis* CICARIA strain.

### Example 3: Primer Validation PCR at Optimal Temperature

For the primers finally selected in Example 2, a primer validation experiment was performed through PCR at an optimal temperature using total DNA, including genomic DNA (gDNA) and plasmid DNA (pDNA) of the target strain and comparative strains, as templates. Sequences of the primer pair are shown in Table 2.

**[Table 2]**

| **Target Strain** | **Direction** | **Sequence (5'->3')** | **SEQ ID NO.** | **Annealing temperature** |
|---|---|---|---|---|
| *Staphylococcus epidermidis* CICARIA | Forward | AATAGCCAAGCATCAAATGCCAG | 2 | 67 °C |
| | Reverse | GGAATTATGTCCCAGGCCCTTT | 3 | |

### Example 4: Confirmation of Specific Reaction of Primers

Experiments were performed to confirm whether the finally selected primers specifically bind only to the target strain.

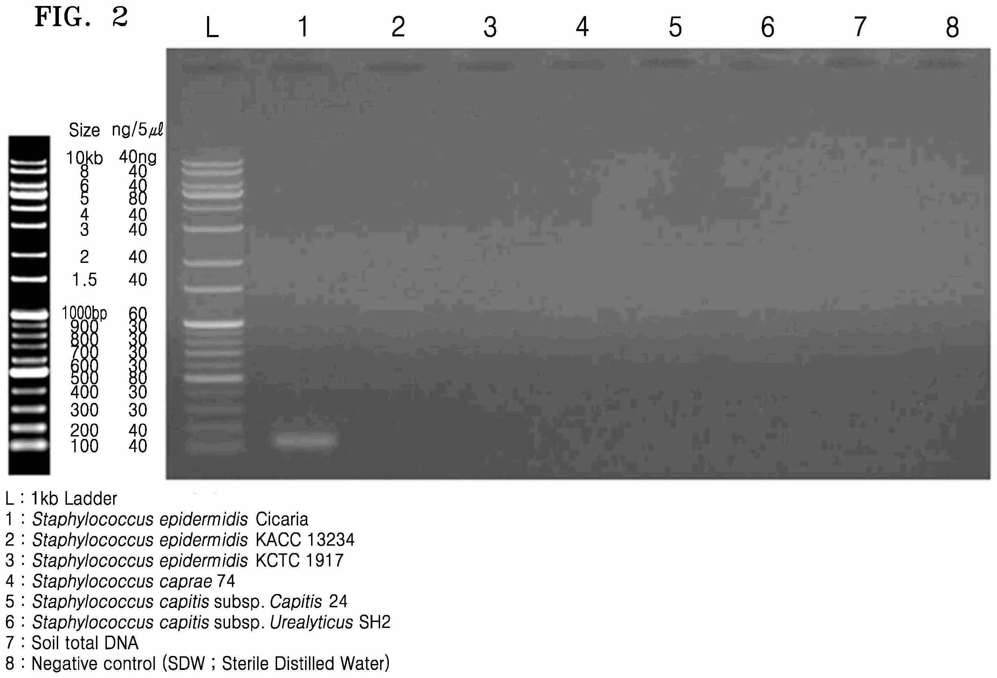

Specifically, PCR was performed using the corresponding primer pair on respective total DNA of *Staphylococcus epidermidis* CICARIA, *Staphylococcus epidermidis* KACC13234, *Staphylococcus epidermidis* KCTC1917, *Staphylococcus caprae 74, Staphylococcus capitis* subsp. *capitis* 24, and *Staphylococcus capitis* subsp. *urealyticus* SH2; environmental sample DNA; and a negative control. Total DNA from soil was used as the environmental sample DNA. Sterile distilled water (SDW) was used as the negative control. Electrophoresis results of the PCR products are shown in FIG. 2.

As a result, as shown in FIG. 2, a 122 bp DNA band was confirmed only in the PCR product obtained using *Staphylococcus epidermidis* CICARIA as a sample.

Optimal PCR amplification conditions for detecting the *Staphylococcus epidermidis* CICARIA strain are shown in Table 3.

**[Table 3]**

| | **Temperature** | **Time** | **Cycles** |
|---|---|---|---|
| Pre-denaturation | 95 °C | 3 minutes | 1 cycle |
| Denaturation | 95 °C | 30 seconds | 30 cycles |
| Annealing | 67 °C | 30 seconds | |
| Extension | 72 °C | 30 seconds | |
| Final Extension | 72 °C | 3 minutes | 1 cycle |
| Hold | 4 °C | - | - |

## Claims

1. A composition for detecting a *Staphylococcus epidermidis* CICARIA strain deposited under Accession No. KCCM12559P, the composition comprising an agent capable of specifically detecting a polynucleotide consisting of a sequence at positions 3,487 to 3,608 of SEQ ID NO: 1 or complementary polynucleotide thereof.

2. The composition of claim 1, wherein the polynucleotide or complementary polynucleotide thereof is a partial sequence of plasmid DNA of the *Staphylococcus epidermidis* CICARIA strain.

3. The composition of claim 1, wherein the agent is a primer or probe that specifically binds to the polynucleotide or complementary polynucleotide thereof.

4. The composition of claim 3, wherein the primer is a primer set consisting of a forward primer set forth in SEQ ID NO: 2 and a reverse primer set forth in SEQ ID NO: 3.

5. A primer set for detecting a *Staphylococcus epidermidis* CICARIA strain deposited under Accession No. KCCM12559P, consisting of a forward primer set forth in SEQ ID NO: 2 and a reverse primer set forth in SEQ ID NO: 3.

6. A kit for detecting a *Staphylococcus epidermidis* CICARIA strain deposited under Accession No. KCCM12559P, comprising the composition of claim 1.

7. The kit of claim 6, further comprising reagents required for a nucleic acid amplification reaction.

8. A method of detecting a *Staphylococcus epidermidis* CICARIA strain deposited under Accession No. KCCM12559P, comprising: isolating nucleic acids from a sample;
performing a nucleic acid amplification reaction using the nucleic acids as templates and the primer set of claim 5; and
detecting an amplification product generated by the nucleic acid amplification reaction.

9. The method of claim 8, wherein the nucleic acids are plasmid DNA, or a mixture of plasmid DNA and genomic DNA.

10. The method of claim 8, wherein when an amplification product having a length of 122 bp is detected, the sample is identified as the *Staphylococcus epidermidis* CICARIA strain deposited under Accession No. KCCM12559P, or the strain is identified as being present in the sample.
